# EUROPEAN PATENT APPLICATION

(11) **EP 3 144 382 A1**
(43) Date of publication of application: **22.03.2017**
(21) Application number: 15793414.2
(22) Date of filing: 12.05.2015
(51) Int. Cl.: C12N 1/20, C12N 1/00

(54) **METHOD FOR PRODUCING LACTIC ACID BACTERIA MEDIUM, METHOD FOR CULTURING LACTIC ACID BACTERIA USING SAME, AND LACTIC ACID BACTERIA POWDER USING LACTIC ACID BACTERIA OBTAINED BY SAID CULTURE METHOD**

(30) Priority: 15.05.2014 JP 2014101133
(71) Applicant: Ebara Foods Industry, Inc., Yokohama-shi, Kanagawa 220-0012 (JP)
(72) Inventor: UENO Shigenori, Yokohama-shi Kanagawa 220-0012 (JP); OHASHI Atsushi, Yokohama-shi Kanagawa 220-0012 (JP)
(74) Representative: D Young & Co LLP
(86) International application number: PCT/JP2015/063612
(87) International publication number: WO 2015/174407

(57) **Abstract**

The following are provided according to the present invention: a method for producing a medium that can be applied to different types of bacterial species and strains for, in particular, lactic acid bacteria culture, such medium being capable of achieving high density of bacterial cells and endowing stability such as heat tolerance and active proliferative capacity within low-temperature and high-temperature ranges to bacterial cells; and a method for culturing bacterial cells using such medium. In a mixture that constitutes a liquid medium, carbonates and germ koji are mixed and sterilized while the other mixture is separately sterilized. The other mixture containing a germ koji extract, different nitrogen and carbon sources, animal or plant fat or oil containing oleic acid, polysorbate, and iron is mixed to complete the medium. Bacterial cells cultured in such medium have excellent heat tolerance and bacterial cell stability, and therefore, the cells can be formed into a dry powder at a good yield.

## Description

### Technical Field

The present invention relates to a method for producing a liquid medium suitable for high-density culture of lactic acid bacteria (rod-shaped lactic acid bacteria and coccus-shaped lactic acid bacteria). In particular, the present invention relates to a method for producing a liquid medium that can be applied to a variety of bacterial species and strains having different properties and can achieve high bacterial cell density in a culture solution, thereby allowing bacterial cells to achieve stability in terms of heat tolerance, acid tolerance, salt tolerance, or the like and/or active proliferative capacity in both low-temperature and high-temperature ranges. The present invention also relates to a method for culturing lactic acid bacteria using such liquid medium and a lactic acid bacteria powder obtained from lactic acid bacteria cultured by the above culture method.

### Background Art

### <Health functionalities of lactic acid bacteria>

The term "lactic acid bacteria" collectively refers to a group of bacteria capable of producing large amounts of lactic acid. Lactic acid bacteria are involved in production of alcoholic beverages, brewed products, pickles, processed fruit products, and the like, in addition to various fermented foods. Thus, it is clear that humans have been benefiting from lactic acid bacteria since ancient times. In recent years, lactic acid bacteria have been gaining attention because they exhibit various health functionalities, such as, by inhibiting intestinal putrefaction bacteria, regulating the bowel movements, and exerting immunostimulatory action (Non-Patent Literature 1).

Lactic acid bacteria as well as bifidobacteria are known as effective intestinal bacteria for human health maintenance. There is a marked upswing in research on "probiotics" guaranteed to be safe and containing microorganisms that improve intestinal flora for health benefits. Against the background of a growing interest in lifestyle-related diseases, the pursuit of such highly desirable health functionalities in food is a growing trend. Furthermore, there is also demand for probiotics that can be effectively used in foods under such circumstances (Non-Patent Literature 2).

### <Trends in methods for culturing lactic acid bacteria>

Meanwhile, one of the currently popular methods for culturing lactic acid bacteria is the batch-wise culture method, which comprises inoculating a liquid medium with a microorganism such as a lactic acid bacterium, performing culture while controlling the medium pH and the like, and then separating and collecting bacterial cells from the culture solution of the stationary growth phase during which cell growth is arrested. In accordance with such method, proliferation of lactic acid bacteria is likely to be inhibited during bacterial growth because the medium environment changes due to a decrease in the amounts of nutrients in the medium and an increase in the amounts of secretion products such as lactic acid during culture. As a result, the bacterial cell density is unlikely to reach 10 billion cfu (colony forming units) or more per 1 ml of culture solution, and bacterial cells may fail to achieve stress resistance. Therefore, techniques that can be employed to increase the density of bacterial cells and simultaneously endow even higher degrees of stability heretofore have been awaited (Non-Patent Literature 3).

In the case of liquid culture medium, it is relatively easy to prepare a medium at low cost. In addition, bacterial cells and secreted substances are likely to be suspended in the culture medium, thereby readily ensuring homogeneity of the culture solution. Further, treatment such as stirring, shaking, or medium pH control during culture can be readily carried out with certainty. For example, there is a report that a bacterial strain of *Lactobacillus plantarum* was subjected to shaking culture at 37°C for 24 hours in a liquid medium containing wheat distilled waste supplemented with nutrients such as glucose and molasses while controlling pH with ammonium hydroxide at 6.0, thereby achieving a bacterial cell density of 1.6 billion cfu/ml at a maximum (Non-Patent Literature 4). As the liquid medium used in this report is inexpensive, such medium is expected to contribute to reduce the product cost. Meanwhile, it has been obviously not considered whether the liquid medium has an effect of realizing high cell density of different bacterial species having widely varying properties or whether the liquid medium can alter properties of bacterial cells by, for example, endowing excellent stability to bacterial cells during high-density liquid culture. In addition, since it is possible to allow lactic acid bacteria of any bacterial species to proliferate using a medium rich in nutrients such as a commercially available liquid medium (MRS: de Man, Rogosa, Sharpe), efficiency of the liquid medium culture has been considered good. However, lactic acid bacteria prepared in such artificial culture environment that totally differs from a natural habitat are disadvantageous in that they are likely to have insufficient survival ability, instead of their original excellent survival ability. Regarding solutions for the above problem, there are many reports on techniques for applying stress stimulation to bacterial cells via, for example, heat treatment, low-temperature treatment, or acid treatment so as to allow the bacterial cells to exert their original excellent stress resistance during main culture, i.e., the final stage of extended culture (Non-Patent Literature 5). However, it is still difficult to achieve high density of bacterial cells in a culture solution or to obtain bacterial cells that exert the original excellent stability and active proliferative capacity. That is, there have been no reports concerning the culture methods that can be used as "bacterial strain breeding."

It is known that when a lactic acid bacterium having poor survival ability is used as a starter culture for preparing fermented foods, such as kimchi, a microbial flora exchange phenomenon is induced during the course of preservation of the food, resulting in death or reduction of the starter bacterial cells even if the starter bacterial strain inherently has excellent health functionalities. In such case, excellent health functionalities derived from the starter bacterial strain cannot be maintained in such a fermented foods. Therefore, selective culture of a lactic acid bacterium that has a high level of survival ability and therefore does not cause such microbial flora exchange phenomenon has been awaited (Non-Patent Literature 6).

### <Difference in the optimal pH range of a culture environment >

In view of the above, a method for culturing *Enterococcus faecalis* or *Enterococcus faecium* via stirring culture, comprising using a mixture containing one or more members selected from the group consisting of magnesium carbonate, monosaccharide or disaccharide, peptone, koji, koji extract, germ medium, yeast, and yeast extract for a liquid medium, has been proposed (Patent Literature 1). According to such culture method, the initial pH of a medium is adjusted to an alkaline level of about pH 8.0. As a result, the medium is allowed to obtain a high level of pH buffering capacity within a pH range of 5.0 to 6.0 so as to prevent conversion of lactic acid produced during culture into non-dissociated form and self-induced cell damage during culture. This allows efficient production of bacterial cells of the above species having excellent resistance to an alkaline pH range inherently in a liquid batch-wise culture system at low cost in an industrial manner. However, it has been impossible to apply the culture method to most of other bacterial species and strains, which have the character of low resistance to an alkaline pH range, compared with the above mentioned species. Further, as disclosed in claim 5 of Patent Literature 1, residual carbonates which are low solubilities in the culture solution at the end of culture, and therefore, it should be dissolved by adding acidic reagent to adjust the culture pH to 4-6 so as to dissolve all carbonates. In view of this, it has been presumably difficult to adapt low-pH stress, thereby failing to endow excellent stability. This is obvious from the fact that the level of heat tolerance of bacterial cells obtained by the culture method disclosed in Test Example 10 in Patent Literature 1 (17% to 26%) was almost the same level of that of bacterial cells obtained from the control cultured in a commercially available BL liquid medium (23% to 30%). Further, Patent Literature 1 discloses that only bacterial species having excellent stability inherently were used in the Examples. Therefore, it has been impossible to conceive of obtaining a finding that stability can be further improved by modifying or improving a culture method for achieving high density of bacterial cells. In addition, the method disclosed in Patent Literature 1 was not a culture method for altering properties of bacterial cells by, for example, endowing active proliferative capacity. This is suggested by the fact that bacterial cells prepared by liquid culture for the achievement of high cell density disclosed in Non-Patent Literature and the like were not used as inocula to be inoculated into the liquid media in the Test Examples and Examples in Patent Literature 1. Accordingly, it is obvious that the culture method of Patent Literature 1 lacks factors of "bacterial strain breeding."

### Citation List

### Patent Literature

Patent Literature 1: JP Patent No. 3900484

### Non-Patent Literature

Non-Patent Literature 1: Ali AA, "Beneficial role of lactic acid bacteria in food preservation and human health: a review," Research Journal of Microbiology, 5(12), pp. 1213-1221 (2010)
Non-Patent Literature 2: Naagpal R, Kumar A, Kumar M, Behare PV, Jain S, Yadav H, "Probiotics, their health benefits and application for developing healthier foods: a review," FEMS Microbiology Letters, 334(1), pp. 1-15 (2012)
Non-Patent Literature 3: Lacroix C, Yildirin S, "Fermentation technologies for the production of probiotics with high viability and functionality," Current Opinion in Biotechnology, 18, pp. 176-183 (2007)
Non-Patent Literature 4: Krzywonos M, Eberhard T, "High density process to cultivate Lactobacillus plantarum biomass using wheat stillage and sugar beet molasses," Electronic Journal of Biotechnology, 14(2), (2011) http://dx.doi.org/10.2225/vol14-issue2-fulltext-10
Non-Patent Literature 5: Serrazanetti DI, Gottardi D, Montanari C, Gianotti A, "Dynamic stresses of lactic acid bacteria associated to fermentation process," Lactic Acid Bacteria-R & D for Food, Health and Livestock Purposes. Intech, Chapter 23, pp. 539-570 (2013)
Non-Patent Literature 6: Lee SK, Ji GE, Park YH, "The viability of bifidobacteria introduced into kimchi," Letters in Applied Microbiology, 28, pp. 153-156 (1999)
Non-Patent Literature 7: Sawatari Y and Yokota A., "Diversity and mechanisms of alkali tolerance in Lactobacilli," Applied and Environmental Microbiology, 73(12), pp. 3909-3915 (2007)
Non-Patent Literature 8: Takashi Sasaki, Takashi Morishita, Mariko Kadota: Genetics and Breeding of Lactic Acid Bacteria 3, Gene transfection and mutagenesis, "Science and Technology of Lactic Acid Bacteria," Japanese Study Group for Lactic Acid Bacteria (eds.) (Gakkai Shuppan Center), pp. 152-159(1996)
Non-Patent Literature 9: Silva J, Carvalho AS, Ferreira R, Vitorino R, Amado F, Domingues P, Teixeira P, Gibbs PA, "Effect of the pH of growth on the survival of Lactobacillus delbrueckii subsp. bulgaricus to stress conditions during spray-drying," Journal of Applied Microbiology, 98, pp. 775-782 (2005).

### Summary of Invention

### Technical Problem

### <Explanation of high-density culture>

Our aim of the present invention is to provide a method for high-density culture of lactic acid bacteria. Such culture method comprises applying various forms of stress stimulation to bacterial cells over the course of the growth phase. This growth phase ranges from the lag phase to the logarithmic phase, and it also includes the stationary phase in both the preculture stage for preparing an inoculum used in main culture and the final stage for main culture. This allows a given bacterial strain to exhibit its inherently optimal environmental adaptability, that is acclimation. The result of this is expected to increase the culture efficiency and improve the bacterial cell density. The culture method also serves as a "bacterial strain breeding" method. With the use of such culture method, the bacterial cell density can reach a level of 10 billion cfu or more per 1 ml of culture solution depending on bacterial species and strains. These figures significantly exceeds the bacterial cell densities achievable with methods that do not involve high-density culture, which are on the order of about 1 billion cfu/ml. The aforementioned higher densities contribute to the reduction of bacterial cell production costs. In addition, excellent stability can be endowed in terms of heat tolerance, active proliferative capacity in low-temperature ranges, and the like, resulting in improved bacterial cell properties. In this regard, the method of the present invention is also characterized in that it serves as a "bacterial strain breeding" method, contributing to more rapid production of fermented food. In addition, the method is expected to result in higher-quality food product features, such as reduced salt content and extended expiration dates. Moreover, the method is expected to improve probiotics in relation to the bacterial cell production steps, product storage period, and resistance capability against stress factors in the intestinal tracts of humans, livestock, pet animals, and the like. Thus, the method is also expected to further boost the health functionalities exhibited by such bacterial strains.

### <Versatility>

The optimum pH range for culture widely varies among lactic acid bacteria depending on bacterial species and strains (Non-Patent Literature 7). Therefore, it has been impossible to carry out high-density culture of many bacterial species and strains inferior in resistance to *Enterococcus faecalis* or *Enterococcus faecium* having specifically excellent resistance in an alkaline pH range by the conventional technique disclosed in Patent Literature 1. In view of this, a method for producing a liquid medium that can be applied to a wider variety of bacterial species and strains of lactic acid bacteria has been awaited. Meanwhile, the conventional technique for improving various properties of bacterial cells involves the following three approaches of "bacterial strain breeding:" (1) acquisition of a bacterial mutant strain that has experienced a permanent genetic change (spontaneous mutation or induced mutation); (2) introduction of a foreign gene (transduction, conjugational transfer, cell fusion, or transformation); and (3) omission of some genes (curing of plasmids or prophages) (Non-Patent Literature 8). That is, there have been no reports on culture methods by which high density of bacterial cells can be achieved and various properties of bacterial cells can be improved at the same time.

In order to develop a novel liquid medium that can be applied to different bacterial species and strains of lactic acid bacteria, it is necessary to add nutrients essential for proliferation that are specific to bacterial species and strains, taking into consideration the original natural habitats of such bacteria. It is also necessary to adjust the medium pH, osmotic pressure, and the like in the relevant medium so that bacterial cells will be able to adapt the high stress intensity of the culture environment. Therefore, it is necessary to maintain medium pH within the optimum pH range, which is different for various bacterial species and strains. Some lactic acid bacterial species and strains have very low levels of resistance capability within an alkaline pH range, and therefore, it is necessary to establish the initial medium pH within an acidic range (i.e., about pH 6.0 to 7.0) (Non-Patent Literature 7). Further, as culture proceeds, the pH of the culture solution readily decreases due to lactic acid production, resulting in an increased amount of non-dissociated lactic acid. This causes self-induced cell damage, which is problematic.

As a result of intensive studies to reduce the initial liquid medium pH in view of the above problems, the present inventors achieved a method for maintaining the initial medium pH at a level tolerable for a given bacterial strain to be inoculated. This was accomplished by reducing the amount of magnesium carbonate added and adding calcium carbonate to result in an appropriate compounding ratio for both components. Further, the present inventors found that when germ koji is added to a liquid medium containing appropriate amounts of poorly soluble alkaline carbonates such as calcium carbonate and magnesium carbonate for the purpose of promoting bacterial cell proliferation, and is sterilized together with poorly soluble alkaline carbonates while other medium components are separately sterilized, the sustained release of poorly soluble alkaline carbonates in the medium becomes possible. By culturing lactic acid bacteria using a medium comprising a mixture of such sterilized germ koji and poorly soluble alkaline carbonates, it becomes possible to reduce the initial pH of the medium and slow down the rate of pH decrease resulting from the production of organic acids such as lactic acid produced during culture. As described above, the improvement of the pH buffering capacity of the liquid medium of the present invention results in prevention of conversion of lactic acid produced during culture to non-dissociated lactic acid, which is strongly toxic to bacterial cells. This is expected to be effective for the reduction of self-induced cell damage.

In addition, by carrying out culture in the above medium environment, it becomes possible not only to achieve high bacterial cell density but also to reduce the degree of final pH decrease of a culture solution such that it falls within a pH range that is both necessary and sufficient to endow excellent stability in terms of heat tolerance and acid tolerance (with the adaptation to acidic pH stress). Further, excellent proliferative capacity within both low-temperature and high-temperature ranges is endowed to bacterial cells, which is effective for the improvement of various properties thereof. Thus, it has been revealed that various properties of bacterial cells can be improved. This has led to the completion of the present invention.

### Solution to Problem

Specifically, according to the method for producing a liquid medium for lactic acid bacteria of the present invention, manganese, heme iron, organic acids such as acetic acid and citric acid, and fat or oil such as olive oil are emulsified with a surfactant polysorbate and then added as nutrients essential for proliferation of many lactic acid bacteria other than *Enterococcus faecalis* and *Enterococcus faecium* disclosed in Patent Literature 1 to a liquid medium in order to improve medium components. Further, if a natural habitat for lactic acid bacteria is a symbiotic system containing yeast, it is also necessary to supplement dry yeast cells. The method of the present invention is a method for producing a liquid medium for lactic acid bacteria comprising a mixture containing at least poorly soluble alkaline carbonates and germ koji, such method comprising mixing poorly soluble alkaline carbonates and germ koji, sterilizing the mixture while separately sterilizing the other medium components, and then mixing the mixture with the other separately sterilized medium components to complete the liquid culture medium. In such case, a mixture of calcium carbonate and magnesium carbonate can be used as poorly soluble alkaline carbonates. Furthermore, the effects of the present invention can be further enhanced by adding dry yeast cells and increasing the proportion of insoluble components added with respect to the amount of carbonates added during sterilization.

Hereafter, the medium of the present invention is referred to as "high-density liquid medium" and culture using such medium is referred to as "high-density liquid culture."

The composition of a high-density liquid medium may comprise at least one member selected from the group consisting of germ koji extract, a nitrogen source, and a carbon source, in addition to poorly soluble alkaline carbonates and germ koji. Further, other fatty acids, polysorbates serving as surfactants, and iron can be added. In such case, animal and plant fats and oils including oleic acid can be used as fatty acids. In addition, heme iron can be used as iron.

### Advantageous Effects of Invention

When a high-density liquid medium containing germ koji, which is obtained by the method for producing a liquid medium for lactic acid bacteria of the present invention, is used, it allows the sustained release of poorly soluble alkaline carbonates in the medium. Accordingly, such carbonates are neutralized in a manner proportional to the amount of lactic acid produced between the logarithmic growth phase and the stationary growth phase of lactic acid bacteria, thereby preventing a sharp decrease in pH, which is inappropriate for culture. Therefore, it becomes possible to provide a culture medium appropriate for many types of bacterial species and strains having poor alkaline pH resistance, for which it is required to adjust the initial medium pH within a weak acidic range. When the medium is applied to a multi-stage culture, the inoculum that is inoculated into a high-density liquid culture at the initial stage is a seed culture of bacterial cells prepared via static culture in an MRS liquid medium supplemented with 1% light calcium carbonate. In the case of the high-density liquid culture of the present invention, the final pH of the culture solution decreases to about 5.0 during the stationary growth phase. As a result, poorly soluble alkaline carbonates are sufficiently dissolved and thus do not precipitate. In addition, there is an adequate difference between the initial pH and the final pH, which is sufficient for the adapation of acid stress. This is expected to be effective in improving both heat tolerance and acid tolerance.

In addition, an increase in the density of bacterial cells in a culture solution results in an increase in the amount of lactic acid secreted and produced, which allows poorly soluble alkaline carbonates to be converted into water-soluble salts while emitting carbon dioxide. Accordingly, the ion intensity in the culture solution gradually increases, thereby making it possible to apply osmotic pressure stress to bacterial cells. This is expected to be effective in increasing the types of stress applied that are necessary for the improvement of properties of bacterial cells during high-density liquid culture. An increase in ion intensity of the culture solution can be evaluated by determining electrical conductivity. Meanwhile, carbon dioxide emitted in the culture solution is effective in reducing dissolved oxygen, thereby decreasing the production of hydrogen peroxide. This means that even when the bacterial cell density becomes high, the production of hydrogen peroxide does not increase in proportion to the increase in the bacterial cell density; rather, the concentration is appropriately controlled. Therefore, it is possible to maintain the degree of oxidative stress derived from hydrogen peroxide at a desirable level, thereby making it possible to endow resistance against oxidative stress and prevent self-induced cell damage caused by excessive hydrogen peroxide during culture. As described above, by appropriately and gradually applying various forms of stress stimulation that are acceptable for the specific bacterial strains to be cultured, it becomes possible not only to achieve high bacterial cell density but also to selectively culture bacterial cells having optimal stability and active proliferative capacity within both low-temperature and high-temperature ranges, in contrast to culture in a simple medium rich in nutrients, such as an MRS liquid medium.

Further, the present inventors found that if a high-density liquid medium is used not only in main culture that is the largest-scale culture at the final stage of extended culture of lactic acid bacteria with the use of a high-density liquid medium, but also at each stage of preculture or culture prior to preculture in which an inoculum used for main culture is prepared, it becomes possible to achieve high density of bacterial cells and obtain a culture solution containing bacterial cells having bacterial cell stability and active proliferative capacity within low-temperature and high-temperature ranges. This has led to the completion of the present invention. That is, according to the present invention, in order to prepare bacterial cells exhibiting sufficient environmental adaptability that is inherent to their original strain, the target level of such environmental adaptability can be achieved with improved efficiency and certainty by setting the intensity of stress stimulation that is applied at each stage of culture prior to preculture to a relatively low level and increasing the intensity of stress stimulation that is applied to a medium at the subsequent culture stage.

Alternatively, the above high-density liquid medium is used at each stage of the multi-stage extended culture of lactic acid bacteria, thereby making it possible to achieve high density of bacterial cells and culture lactic acid bacteria having bacterial cell stability and active proliferative capacity within low-temperature and high-temperature ranges.

Furthermore, heat tolerance of lactic acid bacteria collected by the above culture method is comparable to heat tolerance of lactic acid bacteria obtained by general culture methods. Therefore, it is possible to obtain a powder of lactic acid bacteria having bacterial cell stability and active proliferative capacity within low-temperature and high-temperature ranges at a good yield even after heat drying such as spray drying using a spray dryer or air drying other than freeze-drying.

### <Definition of terms: Mechanism of achieving high density and stability of bacterial cells>

According to the present invention, preferable "carbonates" are poorly soluble alkaline carbonates such as calcium carbonate and magnesium carbonate. In addition, in order to realize optimum medium pH, it is preferable to mix calcium carbonate and magnesium carbonate at an appropriate compounding ratio and add the mixture to a medium. Except for Patent Literature 1, there have been no reports on the use of magnesium carbonate in a liquid medium for lactic acid bacteria. This is because when magnesium carbonate is added to a liquid medium, the medium pH shifts to the alkaline side from the usual pH (6.0 to 7.0) which is acceptable for many types of lactic acid bacteria. For batch-wise liquid culture, it is desirable for a medium to have increased pH buffering capacity. For such purpose, one idea is to increase the amount of carbonates added. However, in such case, as the initial pH shifts to the alkaline side (pH increase), stringent stress is applied to bacterial species and strains, which have insufficient resistance to alkaline pH (alkaline pH stress). Therefore, if bacterial cells do not have active proliferative capacity at the inoculum preparation stage, the cells might be unable to proliferate. Meanwhile, when sugars including glucose and other medium components are mixed and sterilized together, browning reaction (Maillard reaction) of a medium is promoted. This probably results in production of hydrogen peroxide, leading to inhibition of bacterial proliferation. As a result, the present inventors have conceived of the method for preparing a medium of the present invention, comprising mixing poorly soluble alkaline carbonates and germ koji and treating the mixture via high-temperature and high-pressure sterilization separately from other medium components, thereby decreasing the initial pH of the medium and allowing the carbonates to be slowly released in the liquid medium so as to prevent a sharp pH change in the medium pH.

The poorly soluble alkaline carbonates react with an organic acid so as to be converted into water-soluble salts when the concentration of an organic acid such as lactic acid or acetic acid increases, such organic acid being produced by bacterial cells during culture between the logarithmic growth phase and the stationary growth phase. In addition, carbon dioxide is emitted in the culture solution, resulting in increased ion intensity in the culture solution. This leads to the application of osmotic pressure stress to bacterial cells during culture. Further, stimulation such as alkaline pH stress, acidic pH stress, oxidative stress, or temperature stress is further applied. As a result, the obtained bacterial cells have sufficient stress resistance and improved stability. The term "stress" used herein refers to a culture condition, which does not fall within an optimum range in a culture environment for bacterial species or bacterial strains. Indexes of culture conditions may include pH, osmotic pressure, temperature, the amount of assimilated substances contained in a medium, and the like. As the level of stress deviates from an optimum range, the intensity of stress stimulation increases.

From a plurality of viewpoints, the sustained-release process of the above carbonates also promotes the inhibition of self-induced cell damage caused by lactic acid bacteria themselves to a certain extent. Specifically, carbon dioxide is released into a medium, resulting in reduction of dissolved oxygen in a culture solution. Lactic acid bacteria are facultative anaerobic bacteria and thus they cannot directly use molecular oxygen for energy metabolism. They rather generate hydrogen peroxide and active oxygen such as superoxide radical or hydroxyl radical when coming into contact with oxygen, thereby causing damage to bacterial cells. Therefore, as a result of reduction of dissolved oxygen in a culture solution, an increase in the concentration of hydrogen peroxide or the like is inhibited during culture, thereby preventing self-induced cell damage (1). In addition, when high-density liquid culture is performed, organic acid production capacity is reduced, thereby preventing self-induced cell damage (2). Further, as a result of a reaction of poorly soluble alkaline carbonates with an organic acid such as lactic acid produced by lactic acid bacteria, the rate of reduction of pH in a culture solution is lowered. Accordingly, the rate of non-dissociated lactic acid production is unlikely to increase, thereby inhibiting self-induced cell damage (3).

The mixing ratio of two poorly soluble alkaline carbonates can be appropriately determined depending on the optimum pH of a bacterial strain. However, preferably, at least the amount of magnesium carbonate added does not exceed the amount of calcium carbonate on a weight basis. In addition, when only one of these carbonates is used, the obtained effects would be reduced, which is not preferable.

A plant that is used as a raw material for preparing "germ koji" can be appropriately selected from cereals such as rice, wheat, beans, and corn or defatted germs obtained by removing fats and oils from such cereals. However, in consideration of concern that rancidification of fats and oils takes place during preservation of such raw materials, it is desirable to employ a solid medium composition composed of defatted rice germ, defatted wheat germ, and defatted corn germ or defatted soybeans. In addition, typical examples of microorganisms used for solid-state culture on the surfaces of defatted germ particles include, but are not limited to, *Aspergillus niger,* and *A*. *sojae* as well as *A*. *oryzae.* Examples of "sterilization" of a mixture of poorly soluble alkaline carbonates and defatted germ koji include high-pressure steam sterilization via autoclave sterilization (which is not particularly limited as long as sterilization is performed at 121°C for 15 minutes or more or under similar conditions). The term "germ koji extract" refers to an extract of germ koji (50% ethanol fractionation product or a product obtained by an extraction method which is not limited to such ethanol fractionation). Aspergillus germ fermentation components are added together with germ koji to the liquid medium of the present invention. For example, "rice germ fermentation extract-P" (Oryza Oil & Fat Chemical Co., Ltd.) can be used.

Basic energy sources to be added are a "nitrogen source" and a "carbon sources." Examples of raw material for a nitrogen source include defatted milk powders, soybean peptides, and meat extract. Examples of commercially available products that can be used include polypeptone (Nihon Pharmaceutical Co., Ltd.), HINUTE AM or HINUTE DC6 (Fuji Oil Co., Ltd.), Lab-Lemco powder (Oxoid), and mixtures thereof. Although requirements for carbon sources vary depending on bacterial strains, one or more members selected from the group consisting of glucose, fructose, trehalose, cellobiose, N-acetylglucosamine, mannose, maltose, lactose, and sucrose can be mixed. Note that HINUTE is a decomposed product of soy protein isolate.

The term "fatty acid" refers to a proliferation promoter to be added. Examples of "animal or plant fat or oil containing oleic acid" that can be used include plant oil such as olive oil, beef fat, fish oil, and animal fat. The expression "polysorbate that serves as a surfactant" refers to sorbitan fatty acid ester condensed with about 20 molecules of ethylene oxide. Examples thereof include polysorbate 20, polysorbate 60, polysorbate 65, and polysorbate 80. However, when olive oil mainly comprising unsaturated fatty acid is incorporated into the hydrophobic region of a surfactant for emulsification, polysorbate 80 is particularly preferable. When beef fat is used, polysorbate 20 is preferably used in combination. The term "iron" refers to hem ion that is preferably added as a proliferation promoter for coccus-shaped lactic acid bacteria belonging to the genus such as *Lactococcus.* In addition, when other lactic acid bacteria or bacteria coexisting with fungi in an natural habitat are selected as subjects of culture from among bacterial species and strains of lactic acid bacteria, a liquid medium composition supplemented with nutrients such as citric acid, acetic acid, and dry yeast cells or a peptide fraction (FYE) of yeast cells is appropriately prepared. Dry yeast cells may be cells of either baker's yeast or beer yeast. EBIOS tablets (Asahi Food & Healthcare) were used in the Examples; however, frozen yeast cells (Oriental Yeast Co., Ltd.) may be optionally used.

The term "preculture" refers to culture using an aliquot of a medium, which is carried out as a preliminary step of extended culture. In addition, the liquid medium of the present invention is added in "the steps of the multi-stage culture," resulting in the improvement of properties of bacterial cells. Specifically, in addition to extended main culture with the use of the high-density liquid culture of the present invention, high-density liquid culture is performed to prepare an inoculum with which the liquid medium is inoculated. Accordingly, bacterial cells having high resistance to stress under stringent culture conditions are selectively cultured, which may allow the improvement of stress resistance of bacterial cells having environmental adaptability. For multi-stage culture, it is possible to increase the degree of stringency of culture conditions, that is to say, the degree of stress applied, in a stepwise manner as the culture proceeds.

### Brief Description of Drawings

Fig. 1 shows a graph indicating pH buffering capacity of each medium.
Fig. 2 shows graphs each indicating changes in pH during lactic acid fermentation of soymilk maintained at 4°C or 10°C.
Fig. 3 shows graphs each indicating changes in the amount of lactic acid produced during lactic acid fermentation of soymilk maintained at 4°C or 10°C.

### Description of Embodiments

The method for producing a high-density liquid medium of the present invention and culture of lactic acid bacteria using such medium are described below with reference to the Examples. Although specific bacterial strains are mentioned in the Examples, the scope of the present invention is not limited thereto. The present invention can be applied to a wide range of lactic acid bacteria used in general, including bacterial species of the above bacterial strains.

### [Example 1] <High-density liquid medium components>

A high-density liquid medium containing the components below is prepared. Table 1 shows the amounts of the respective components with respect to the total amount of the medium. Table 2 shows the composition of component (1). Table 3 shows the composition of component (2).

Table 4 shows the composition of component (3). The components (1), (2), and (3) were separately sterilized (via autoclave sterilization at 121°C for 20 minutes). After sterilization, the cooled components were mixed to complete a high-density liquid medium. Each bacterial strain to be cultured was inoculated into such medium.

**(Table 1)**

| | |
|---|---|
| Component (1) | 3.75ml |
| Component (2) | 3.75ml |
| Component (3) | 7.50ml |
| Total | 15.00ml |

**(Table 2)**

| <Component (1)> | |
|---|---|
| Calcium carbonate | 0.30g |
| Magnesium carbonate | 0.12g |
| Defatted germ koji | 0.45g |
| Deionized water | 3.75ml |

**(Table 3)**

| <Component (2)> | |
|---|---|
| Glucose | 0.225g |
| Fructose | 0.225g |
| Trehalose | 0.225g |
| Cellobiose | 0.225g |
| N-Acetylglucosamine | 0.225g |
| Mannose | 0.225g |
| Deionized water | 3.75ml |

**(Table 4)**

| <Component (3)> | |
|---|---|
| Polypeptone (Nihon Pharmaceutical Co., Ltd.) | 0.45g |
| HINUTE AM (Fuji Oil Co., Ltd.) | 0.45g |
| Lab-Lemco powder (Oxoid) | 0.30g |
| Manganese chloride tetrahydrate magnesium | 0.0075g |
| Magnesium glycerophosphate | 0.075g |
| Sodium acetate | 0.03g |
| 36% defatted germ koji extract (50% ethanol fractionation product) | 0.83ml |
| EBIOS tablets (Asahi Food & Healthcare) | 0.15g |
| Heme iron solution (obtained by dissolving protoporphyrin IX (Sigma-Aldrich) serving as heme iron in a 0.05N sodium hydroxide solution (0.5 mg/ml)) | 0.30ml |
| Olive oil | 0.0125g |
| Polysorbate 80 | 0.025g |
| Deionized water | 7.50ml |

### <Culture results of high-density liquid culture>

Table 5 shows results of the number of bacterial cells and bacterial cell stability (heat tolerance, salt tolerance, survival rate after freezing and thawing) which were obtained by preparing inocula using the high-density liquid medium obtained in Example 1 for strains of rod-shaped lactic acid bacteria (*Lactobacillus plantarum* NRIC 146 and *L. rhamnosus* GG (ATCC 53103)) and coccus-shaped lactic acid bacteria (*Pediococcus pentosaceus* ATCC43200 and *P. acidilactici* ATCC 25740) and performing main culture. For control examples, each bacterial strain was cultured in an MRS liquid medium for main culture. In the Examples and control examples, the inocula were prepared under the same conditions (culture temperature: 27°C; static culture; culture time: 24 hours). In addition, a bacterial solution obtained via culture in an MRS liquid medium supplemented with 1% light calcium carbonate under the above conditions was inoculated into a high-density liquid medium for inoculum preparation in Example 1, and it was directly used for preparation of an inoculum in each control example. For main culture, the culture temperature was set to 30°C and the culture time was set to 24 hours. In the Examples, shaking culture was performed while static culture was performed in the control examples. Criteria for the respective indexes of bacterial cell stability are listed in Table 6.

**[Table 5]**

| Strain | Inoculum preparation conditions | | | Medium | | | Turbidity | pH | No. of bacterial cells | Heat tolerance (%) | Salt tolerance (%) | Survival rate after freezing and thawing (%) |
|---|---|---|---|---|---|---|---|---|---|---|---|---|
| | Medium | Temperature | Aeration | Type | Temperature | Aeration | (2N sulfuric acid) | | (× 10^{a} cfu/ml) | (52°C - 15min) | (12% common salt 30°C-2h) | (-30°C) |
| Lactobacillus plantarum | Ca-MRS | 27 | Static culture | MRS | 30 | Static culture | 7.30 | 3.85 | 55.0 | 2.0 | 71.0 | 23.0 |
| (NRIC 146) | High-density liquid | 27 | Static culture | High-density liquid | 30 | Shaking culture | 43.50 | 4.83 | 160.0 | 29.0 | 50.0 | 36.0 |
| Lactobacillus rhamnosus GG | Ca-MRS | 27 | Static culture | MRS | 30 | Static culture | 6.69 | 3.85 | 56.0 | 2.9 | 7.9 | 54.0 |
| (ATCC 53103) | High-density liquid | 27 | Static culture | High-density liquid | 30 | Shaking culture | 39.40 | 5.03 | 260.0 | 58.0 | 77.0 | 58.0 |
| Pediococcus pentosaceus | Ca-MRS | 27 | Static culture | MRS | 30 | Static culture | 3.96 | 4.11 | 8.3 | 35.0 | 64.0 | 64.0 |
| (ATCC43200) | High-density liquid | 27 | Static culture | High-density liquid | 30 | Shaking culture | 32.70 | 4.92 | 150.0 | 60.0 | 110.0 | 87.0 |
| Pediococcus acidilactici | Ga-MRS | 27 | Static culture | MRS | 30 | Static culture | 7.36 | 5.48 | 41.0 | 93.0 | 115.0 | 80.0 |
| (ATCC 25740) | High-density liquid | 27 | Static culture | High-density liquid | 30 | Shaking culture | 36.48 | 500 | 115.0 | 86.0 | 113.0 | 87.0 |

**(Table 6)**

| | |
|---|---|
| Heat tolerance | Survival rate (%) after treatment (52°C for 15 minutes) of a bacterial solution obtained via serial dilution of a culture solution with phosphate buffer (pH 6.8) |
| Salt tolerance | Survival rate (%) after treatment (30°C for 2 hours) at a salt concentration of 12% |
| Survival rate after | Survival rate (%) after thawing of a frozen culture solution obtained after freezing at |
| freezing and thawing | -30°C for several days |
| Turbidity | Value obtained by diluting a culture solution using 2N-sulfuric acid to adjust absorbance at 660 nm to about 0.5 and multiplying the absorbance by a dilution rate |

As shown in Table 5, the turbidity and the number of bacterial cells achieved remarkably increased for each bacterial strain. For rod-shaped lactic acid bacteria (*L. plantarum* NRIC146 and *L. rhamnosus* GG (ATCC53103)), the number of bacterial cells achieved increased 2.9-fold and 4.6-fold, respectively. Meanwhile, for coccus-shaped lactic acid bacteria (*P. pentosaceus* ATCC43200 and *P. acidilactici* ATCC25740), the number of bacterial cells achieved increased 18-fold and 2.8-fold, respectively. Also, in terms of heat tolerance, there was no significant difference between coccus-shaped lactic acid bacteria (*P. pentosaceus* and P. *acidilactici*), while there was at least a 10-fold difference between the other bacterial strains. There was a significant difference in heat tolerance between the culture solutions of rod-shaped lactic acid bacteria, and the pH difference between high-density liquid culture and MRS liquid culture was as high as about 1.0 or more. In view of this, it was presumed that bacterial cells gained heat tolerance in the Examples because intracellular pH decreased in response to a slow decrease in medium pH, inducing production of molecular chaperones. In addition, as disclosed in Non-Patent Literature 9, medium pH control with continuous titration of alkaline reagent is unlikely to provide such stabilization effects. Substantially comparable or superior results were obtained in the Examples also for other factors of bacterial cell stability (salt tolerance and survival rate after freezing and thawing), compared to the control examples, although such factors varied depending on bacterial strains.

### [Example 2] <Influence of a high-density liquid medium sterilization method upon pH buffering capacity>

As listed in Table 5, there was a significant difference in pH after main culture between the Examples and the control examples. This difference means a difference in the rate of pH decrease due to a difference in pH buffering capacity of each liquid medium. It is presumed that self-induced cell damage was inhibited in the Examples, contributing to the achievement of high density and bacterial cell stability. The influence of a difference in a sterilization method for high-density liquid medium preparation upon pH buffering capacity was analyzed. The analysis results were described below.

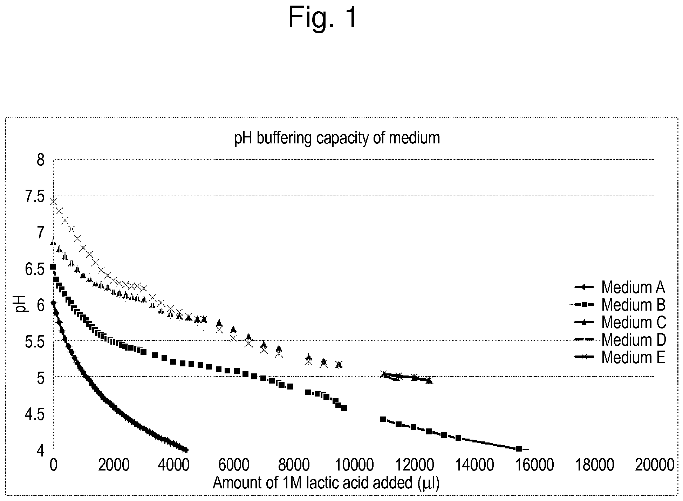

Liquid media (media A, B, C, D, and E) listed in Table 7 were subjected to autoclave sterilization (121°C for 20 minutes) and then 1M concentration lactic acid was added dropwise thereto. The rate of medium pH decrease (degree of pH decrease/the amount of 1M lactic acid added dropwise required for the pH decrease (ml)) was determined (Table 8). Fig. 1 shows a graph indicating time-dependent changes in medium pH for pH buffering capacity of each medium.

**(Table 7)**

| | |
|---|---|
| Medium A(control): | MRS liquid medium |
| Medium B(control): | MRS liquid medium supplemented with 1 % light calcium carbonate |
| Medium C: | Fixture containing the components (1), (2), and (3), which were separately sterilized and cooled |
| Medium D: | Fixture containing the component (2) in Table 1, which was separately sterilized and cooled |
| Medium E: | Medium prepared by adding defatted germ koji to the component (3) during sterilization and separately sterilizing carbonate alone for the component (1) when the components (1), (2), and (3) in Table 1 were separately sterilized |

**(Table 8)**

| | | Amount of lactic acid added to result in pH shown below (ml) | |
|---|---|---|---|
| Medium | pH after sterilization | pH6.0 | pH6.0~pH5.0 |
| Medium A | 6.02 | - | 1.10 |
| Medium B | 6.51 | 0.70 | 6.70 |
| Medium C(the present invention) | 6.87 | 3.30 | 12.00 |
| Medium D | 6.85 | 3.30 | 11.50 |
| Medium E | 7.42 | 3.60 | 12.00 |

As shown in Fig. 1, the initial pH was 6.02 after sterilization for medium A (MRS liquid medium). However, pH decreased to 5.0 at a stage in which 1.10 ml of lactic acid was added dropwise, and pH decreased to 4.0 at a stage in which 4.40 ml of lactic acid was added dropwise. Even in the case of medium B prepared by adding light calcium carbonate (1%) to an MRS liquid medium, it was impossible to decelerate the rate of pH decrease within a relatively high pH range (pH 6.0 or more) and a relatively low pH range (from pH6.0 to pH5.0). Meanwhile, in the case of a medium obtained by separately sterilizing a carbonate mixed with defatted germ koji, a carbon source, and other components (medium C), it was possible to decelerate the rate of pH decrease within both a relatively high pH range and a relatively low pH range. For medium D, pH buffering capacity comparable to that of medium C was not observed within a relatively low pH range. Because the initial pH of medium E after sterilization was too high, it was thought that it was difficult to apply it as a liquid culture for the bacterial strains having weak alkaline resistance inherently.

As stated above, the pH buffering capacity and initial pH significantly varied depending on combinations of components sterilized separately. As a result, it was found that a medium having the pH buffering capacity and initial pH appropriate for bacterial culture can be obtained by combining and sterilizing both at least carbonates and germ koji while separately sterilizing the other components.

### [Example 3] <Effects of the addition of germ koji and germ koji extract>

The effects of the addition of germ koji and germ koji extract on achieving high cell density and endowing stability to bacterial cells were evaluated using the method described below.
(Method) *Lactococcus lactis* ATCC 11454 was used as a coccus-shaped lactic acid bacterium and static culture designated as culture prior to preculture was conducted using an MRS medium containing 1% light calcium carbonate at 27°C for 24 hours. A high-density liquid medium (15 ml) having the composition described in Example 1 was inoculated with an aliquot of the culture solution (0.15ml) obtained in culture prior to preculture, followed by static culture at 30°C for 24 hours. This procedure was designated as preculture. Each of the media (15ml) listed in Table 9 was inoculated with an aliquot of the obtained preculture solution (0.15 ml), followed by main culture at 30°C for 24 hours under static culture conditions. In the control examples, an MRS medium containing 1% light calcium carbonate was inoculated with the bacterial strain described above, and culture was conducted as preculture at 27°C for 24 hours under static culture conditions. An MRS liquid medium (15 ml) was inoculated with an aliquot of the obtained preculture solution (0.15 ml), followed by main culture at 30°C for 24 hours under static culture conditions. Each bacterial solution obtained in main culture was analyzed in terms of turbidity (A), pH, the number of bacterial cells achieved, heat tolerance, salt tolerance, and acid tolerance. Table 10 lists the obtained results. The term "acid tolerance" used in Table 10 refers to the survival rate determined after diluting 100-fold a culture solution using a 0.1 M potassium chloride/hydrochloric acid buffer solution (pH 3.0) and incubating the diluted cell suspensions at 37°C for 2 hours.

**(Table 9)**

| | |
|---|---|
| Medium G: | High-density liquid medium (medium composition described in Example 1) |
| Medium H: | Medium G from which defatted germ koji extract was removed |
| Medium I: | Medium G from which both defatted germ koji and defatted germ koji extract were removed |
| MRS: | (control) |

**[Table 10]**

| Medium | Medium composition | | Initial | | After culture | | | | | | | |
|---|---|---|---|---|---|---|---|---|---|---|---|---|
| | | | Turbidity | pH | Turbidity | | pH | | No. of cells achieved | Heat tolerance (%) Salt tolerance (%) | | Acid tolerance (%) |
| | Defatted germ koji | Defatted germ koji extract | | | Measured value | Change (ΔA) | Measured value | Change (ΔpH) | (×10⁸ cfu/ml) | (52°C - 15 min) | (12% common salt 30°C-2h) | (pH3.0 37°C - 2h) |
| Medium G | Added | Added | 8.64 | 7.15 | 20.64 | 12.00 | 5.12 | -2.03 | 72 | 24.0 | 60 | 190 |
| Medium H | Added | Not added | 8.22 | 7.20 | 19.56 | 11.34 | 5.09 | -2.11 | 69 | 2.2 | 43 | 170 |
| Medium I | Not added | Not added | 5.22 | 7.21 | 14-10 | 8.88 | 5.36 | -1.85 | 31 | 0(below the detection limit) | 30 | 130 |
| MRS | - | - | 0.00 | 5.99 | 1.58 | 1.58 | 4.59 | -1.40 | 10 | 18.0 | 0 | 99 |

As shown in Table 10, physicochemical analysis of the initial medium showed that the lowest turbidity and the highest pH were obtained for medium I. In addition, as a result of microorganism analysis after culture, the number of bacterial cells achieved was lowest for medium I. Also, as a result of characteristics analysis of each culture solution, the lowest results were obtained for medium I in terms of heat tolerance and salt tolerance. Further, it was confirmed that the results for a culture solution obtained from medium H were lower than the results for medium G in terms of heat tolerance and salt tolerance. As described above, when defatted germ koji extract was not added (medium H), differences in heat tolerance and salt tolerance were observed. This suggested that defatted germ koji extract is necessary for the endowment of bacterial cell stability. Furthermore, when both defatted germ koji and defatted germ koji extract were not added (medium I), the bacterial cell stability deteriorated, and the number of bacterial cells achieved remarkably decreased. This suggested that defatted germ koji is necessary for the increase of the number of bacterial cells and endowment of bacterial cell stability.

### [Example 4] <Evaluation of organic acid production capacity>

Organic acid production capacity for high-density liquid culture was evaluated by the culture method described below.
(Method) *Lactobacillus rhamnosus* GG (ATCC53103) was used as a rod-shaped lactic acid bacterium and static culture designated as culture prior to preculture was conducted using an MRS medium containing 1% light calcium carbonate at 30°C for 24 hours. A high-density liquid medium (15 ml) having the composition described in Example 1 was inoculated with an aliquot of the solution (0.15ml) obtained in culture prior to preculture, followed by static culture at 30°C for 24 hours. This procedure was designated as preculture. A high-density liquid medium having the composition of the above medium (15 ml) was inoculated with an aliquot of the obtained preculture solution (0.15 ml), followed by static culture at 30°C for 24 hours or by shaking culture at 160 rpm. This procedure was designated as main culture. In the control examples, an MRS medium containing 1% light calcium carbonate was inoculated with the bacterial strain described above, and static culture was conducted as preculture at 27°C for 24 hours. An MRS liquid medium (15 ml) was inoculated with an aliquot of the obtained preculture solution (0.15 ml), followed by static culture at 30°C for 24 hours or by shaking culture at 160 rpm. This procedure was designated as main culture. Table 11 lists time-dependent changes in pH, turbidity (A), the number of bacterial cells achieved, the organic acid concentration, the degree of change in organic acid concentration, and organic acid production capacity of each main culture solution. Note that the expression "ΔA" in Table 11 indicates a difference between turbidity of a culture solution and turbidity of the relevant sterilized medium.

**[Table 11]**

| No. | Medium | Aeration | Time (h) | Medium pH | Turbidity | No. of cells | Organic acid concentration in medium & culture solution | | | Change in medium - organic acid concentration | | | Organic acid producibility | | |
|---|---|---|---|---|---|---|---|---|---|---|---|---|---|---|---|
| | | | | | (2N | achieved | (mg/ml medium - culture solution) | | | (µmol/ml culture solution) | | | | (µmol/ΔA) | |
| | | | | | sulfuric acid) | (×10^{a}cfu/ml) | Lactic acid | Acetic acid | Citric acid | Lactic acid | Acetic acid | Citric acid | Lactic acid | Acetic acid | Citric acid |
| 1 | MRS | Static culture | 16 | 4.13 | 5.03 | 31 | 8.88 | -0.09 | -0.08 | 98.6 | -150 | -0.417 | 19.8 | -0.300 | -0.084 |
| 2 | MRS | Static culture | 22 | 3.94 | 5.50 | 27 | 11.4 | -0.03 | -0.01 | 127 | -050 | -0.052 | 23.3 | -0.091 | -0.010 |
| 3 | MRS | Static culture | 26 | 3.91 | 5.83 | 29 | 11.9 | -0.03 | -0.06 | 132 | -0.50 | -0.313 | 22.7 | -0.066 | -0.054 |
| 4 | MRS | Shaking culture | 16 | 4.02 | 5.85 | 40 | 10.6 | 0.01 | 0.03 | 117 | -1.67 | 0.156 | 2.2 | -0.029 | 0.027 |
| 5 | MRS | Shaking culture | 22 | 3.89 | 6.00 | 45 | 12.2 | 0.05 | 0.04 | 143 | 0.83 | 0208 | 23.9 | 0.140 | 0.035 |
| 6 | MRS | Shaking culture | 26 | 3.85 | 6.05 | 32 | 13.3 | 0.04 | 0.00 | 148 | 0.67 | 0.000 | 24.6 | 0.111 | 0.000 |
| 7 | High density | Static culture | 16 | 5.02 | 21.70 | 160 | 29.9 | 2.68 | -0.85 | 332 | 24.8 | -4.43 | 19.6 | 1.47 | -0262 |
| 8 | High density | Static culture | 22 | 4.79 | 28.30 | 240 | 45.1 | 3.47 | -0.82 | 500 | 57.8 | -4.27 | 21.3 | 2.46 | -0.182 |
| 9 | High density | Static culture | 26 | 4.73 | 3220 | 190 | 49.1 | 3.42 | -0.81 | 544 | 57.0 | -4.22 | 19.8 | 2.08 | -0.154 |
| 10 | High density | Shaking culture | 16 | 4.29 | 32.30 | 160 | 38.0 | 3.52 | -0.35 | 422 | 58.8 | -1.82 | 15.3 | 2.13 | -0.066 |
| 11 | High density | Shaking culture | 22 | 4.81 | 34.80 | 310 | 49.6 | 3.74 | -0.28 | 550 | 62.3 | -1.46 | 18.3 | 2.08 | -0.049 |
| 12 | High density | Shaking culture | 26 | 4.78 | 37.80 | 380 | 52.7 | 3.92 | -0.23 | 585 | 65.3 | -1.20 | 17.7 | 1.98 | -0.036 |
| Initial MRS liquid medium | | | | 6.06 | 0.036 | | 0.25 | 2.33 | 1.57 | | | | | | |
| Initial high-density liquid medium Initial high-density liquid | | | | 7.62 | 4.80 | | 1.14 | 1.14 | 2.51 | | | | | | |
| Molecular weight | | | | | | | 90.08 | 60.1 | 192 | | | | | | |

As shown in Table 11, pH of the culture solution decreased from the initial pH with the elapse of culture time. Although organic acids were produced at high concentrations in the high-density liquid medium, pH of the culture solution was maintained at a level higher than that of the MRS liquid medium. Regarding the organic acid concentration in the culture solution, the concentration of lactic acid increased in the time-dependent manner in both media. An increase in the organic acid concentration for shaking culture was greater than that for static culture. In addition, the organic acid concentration for the high-density liquid medium was several times higher than that for the MRS liquid medium. Further, in addition to lactic acid production, acetic acid production was confirmed for the high-density liquid medium. *L. rhamnosus* GG (ATCC53103) is known as a bacterial strain characterized by "homolactic fermentation" in which only lactic acid is produced as an organic acid during lactic acid fermentation. It was presumed that the alteration of properties of bacterial cells during high-density liquid culture was effective in causing a pathway, in which a precursor of the lactic acid production pathway is partially converted into acetic acid, to be newly activated. Meanwhile, the organic acid production capacity (production of organic acid (µmol) based on an increase in turbidity of the culture solution) for the high-density liquid medium tended to be lower than that for the MRS liquid medium. This was obvious especially during shaking culture. Such inhibition of lactic acid production capacity was considered to be a characteristic particular to high-density liquid culture. This is because as the pathway of lactic acid fermentation of lactic acid bacteria is controlled by the mechanism of "end product inhibition," the mechanism is unlikely to be activated when the lactic acid production during culture is reduced, which would result in the achievement of an increase in the number of bacterial cells.

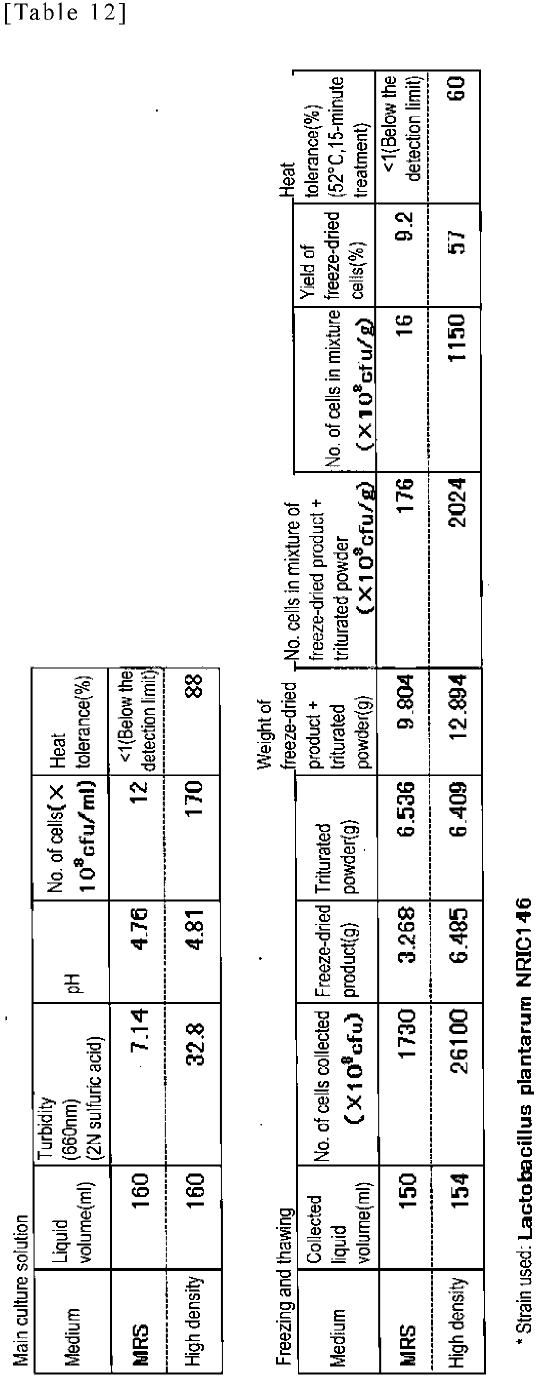

### [Example 5] <Freeze-drying of bacterial cells obtained via high-density liquid culture>

Table 12 shows that the endowment of heat tolerance and the improvement of freeze-drying yield as well as the achievement of high density of bacterial cells can be realized by high-density liquid culture in an environment in which various forms of stress stimulation are applied to bacterial cells.
(Method) *L. plantarum* NRIC146 was precultured in each of an MRS medium and a high-density liquid medium (liquid medium volume: 16 ml) at 30°C and a shaking rate of 165 rpm for 24 hours. An aliquot of each obtained preculture solution (1.6 ml) was inoculated into the relevant liquid medium (liquid volume: 160 ml), followed by main culture (under the same culture conditions for preculture). The number of bacterial cells obtained in main culture was 170 x 10⁸ cfu/ml for the high-density liquid medium, which significantly exceeded 12 x 10⁸ cfu/ml for the MRS medium. The difference in the number of bacterial cells between both media was greater than the difference in turbidity therebetween. In view of this, it was considered that shaking culture caused serious self-induced cell damage in the MRS liquid medium used as a control. This was also suggested by the fact that the level of heat tolerance of bacterial cells in the main culture solution was 88% for the high-density liquid medium, while on the other hand, it was less than the detection limit for the MRS medium. The yield of bacterial cells after freeze-drying was 9.2% for the MRS liquid medium, while on the other hand, it was 57% for the high-density liquid medium. Further, heat tolerance of freeze-dried bacterial cells was substantially comparable to that of bacterial cells in the main culture solution. In view of this, it was confirmed that dry bacterial cells having excellent resistance to bacterial cell damage due to freeze-drying and sufficient heat tolerance were successfully prepared.

**[Table 13]**

| <4°C> | | | | | | | | | | | | | | |
|---|---|---|---|---|---|---|---|---|---|---|---|---|---|---|
| pH | | | | | | | | Lactic acid(mg/100g) | | | | | | |
| No. of days | 0 | 1 | 2 | 5 | 9 | 13 | | No. of days | 0 | 1 | 2 | 5 | 9 | 13 |
| MRS | 6.70 | 6.62 | 6.26 | 6.33 | 5.74 | 5.09 | | MRS | 0 | 22 | 28 | 69 | 175 | 324 |
| High density | 6.70 | 5.56 | 5.18 | 4.47 | 4.24 | 4.35 | | High density | 0 | 160 | 281 | 649 | 771 | 712 |
| | | | | | | | | | | | | | | |

| <10°C> | | | | | | | | | | | | | | |
|---|---|---|---|---|---|---|---|---|---|---|---|---|---|---|
| pH | | | | | | | | Lactic acid(mg/100g) | | | | | | |
| No. of days | 0 | 1 | 2 | 5 | 9 | 13 | | No. of days | 0 | 1 | 2 | 5 | 9 | 13 |
| MRS | 6.70 | 6.20 | 5.48 | 4.51 | 4.40 | 4.48 | | MRS | 0 | 54 | 256 | 620 | 685 | 608 |
| High density | 6.70 | 4.96 | 4.73 | 4.27 | 4.34 | 4.47 | | High density | 0 | 312 | 584 | 803 | 708 | 602 |

### [Example 6] <Evaluation of lactic acid fermentation in soymilk - Endowment of low-temperature proliferative capacity>

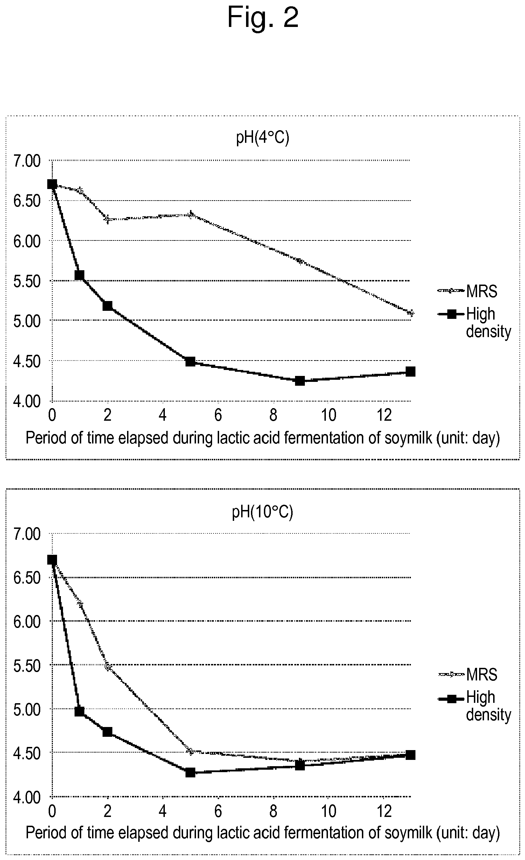

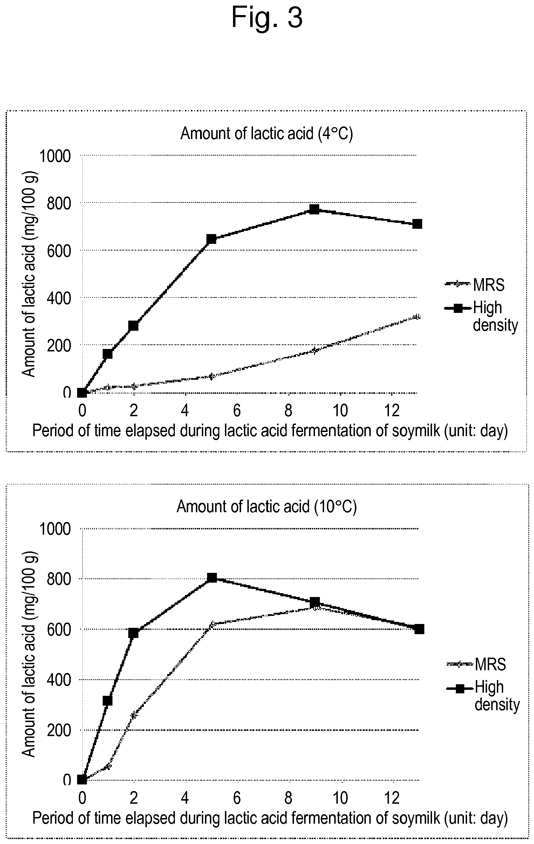

For starter culture, freeze-dried bacterial cells of *Lactobacillus plantarum* NRIC146 obtained in Example 5 were inoculated into commercially available whole soymilk such that the bacterial cell density resulted in 14 x 10⁸ cfu/ml, followed by static culture at 4°C and 10°C for lactic acid fermentation. Accordingly, time-dependent changes in pH and lactic acid concentration were evaluated. Table 13 shows the obtained results and Figs. 2 and 3 show graphs indicating the results. As is apparent from Table 13 and Figs. 2 and 3, freeze-dried bacterial cells obtained via high-density liquid culture immediately started lactic acid fermentation within a low-temperature range, compared with freeze-dried bacterial cells obtained in the MRS liquid medium used as a control, resulting in solidification of soymilk. (The isoelectric point of soybean protein in soymilk is 4.5 and solidification takes place when pH reaches about 4.5.) The degree of vigor of fermentation obviously differed at a lower temperature, which was 4°C. The difference between both media decreased at 10°C as the day went on. However, even 13 days later, there was still about a 2-fold difference in the amount of lactic acid therebetween at 4°C. The above findings concluded that low-temperature proliferative capacity can be endowed via high-density liquid culture.

### [Example 7] <High-temperature proliferative capacity endowed by the application of temperature stress>

It was determined using the culture method described below whether or not high-temperature proliferative capacity could be endowed by applying temperature stress during high-density liquid culture with the use of *Pediococcus acidilactici* ATCC25740 as a coccus-shaped lactic acid bacterium.
(Method) For culture prior to preculture, *P. acidilactici* ATCC25740 was inoculated into an MRS liquid medium supplemented with light calcium carbonate (1%), followed by static culture at 37°C for 20 hours. For preculture, an aliquot of the culture solution (0.15ml)) obtained via culture prior to preculture was inoculated into a high-density liquid medium (9% Glc) and another high-density liquid medium (9% C-Mix) (15 ml each) listed in Table 14, followed by shaking culture at 37°C and 160 rpm for 22 hours. An MRS liquid medium (15 ml) and an MRS liquid medium supplemented with 1% light calcium carbonate (15 ml) were used as controls and inoculated with an aliquot of the culture solution (0.15ml) obtained via culture prior to preculture. For preculture, the former medium was subjected to static culture at 37°C for 22 hours and the latter medium was subjected to shaking culture at 37°C and 160 rpm for 22 hours. Further, for main culture, the aliquots of the aforementioned preculture solution (9% Glc) and the other preculture solution (9% C-Mix) (0.15 ml each) were inoculated into a high-density liquid medium (7% Glc) and another high-density liquid medium (7% C-Mix) (15 ml each) listed in Table 14, respectively, followed by shaking culture at 42°C and 200 rpm for 22 hours. Aliquots of the preculture solutions used as controls (0.15 ml each) were inoculated into an MRS liquid medium and an MRS liquid medium supplemented with 1% light calcium carbonate, same as in each preculture. For main culture, the MRS liquid medium was subjected to static culture at 42°C for 22 hours and the MRS liquid medium supplemented with 1% light calcium carbonate was subjected to shaking culture at 42°C and 200 rpm for 22 hours. For evaluation of heat tolerance, each culture solution was serially diluted with phosphate buffer (pH 6.8) and treated at 57°C for 15 minutes to obtain the survival rate (%) of bacterial cells in the resulting bacterial solution. For evaluation of electrical conductivity, each medium or culture solution was diluted 10-fold with deionized water. The resulting dilution was analyzed using a multi water quality analyzer (MM-60R; DKK-TOA Corporation). The obtained value was multiplied by a dilution rate of 10 and expressed with units of mS/m. Table 15 shows the results obtained for the preculture solutions and the main culture solutions. In the case of high-density liquid culture, the number of bacterial cells achieved increased 1.4- to 1.7-fold when shifting up culture temperature from 37°C to 42°C. Meanwhile, the number of bacterial cells achieved in the case of shaking culture in the MRS liquid medium supplemented with calcium carbonate (1%) was about 1.5 times as large as the figures for static culture in the MRS liquid medium at both 37°C and 42°C. However, the number of bacterial cells achieved in each MRS liquid medium did not increase even when shifting up culture temperature from 37°C to 42°C. In addition, heat tolerance at 57°C in the case of culture in the MRS liquid medium supplemented with calcium carbonate (1%) was lower than that in the case of culture in the MRS liquid medium. High levels of heat tolerance at 57°C were confirmed for the culture solutions obtained via shaking culture in the high-density liquid media. In particular, bacterial cells that were found to have survived after freezing and thawing showed a high level of heat tolerance at 57°C, indicating that sufficient heat tolerance had been endowed through high-density liquid culture. Table 16 shows the results of organic acid production for the respective culture solutions. The organic acid concentrations in the culture solutions of the high-density liquid media were greater than those in the controls at both the preculture stage at 37°C and the main culture stage at 42°C, reflecting high bacterial cell densities in the culture solutions. However, organic acid production capacity, which was evaluated based on bacterial cell turbidity, was lower in the case of high-density liquid culture, compared with the controls, at both culture stages. In addition, the degree of development of a group of enzymes involved in organic acid production was evaluated using washed bacterial cells from each culture solution. Tables 17 and 18 show the results. For evaluation, bacterial cells in each culture solution were centrifuged and collected. The bacterial cells were washed once with phosphate buffer (pH 6.8) and added to 10 ml of a 5% glucose solution (Glc) and 10 ml of 5% D-xylose solution (XYL), such that each resulting solution had a turbidity of 0.3. The resulting solutions were incubated at 30°C for observation of time-dependent changes in pH. The results showed no obvious differences between high-density liquid culture and control culture at the preculture stage at 37°C. On the other hand, there was a large difference at the main culture stage at 42°C, indicating that pH reduction was actively promoted in both of the solutions inoculated with bacterial cells obtained via high-density liquid culture. The above findings suggest that when the culture temperature was increased to 42°C for high-density liquid culture, high-temperature proliferative capacity was endowed and the development of enzymes in the organic acid production system resulted in the modification of properties of bacterial cells, thereby facilitating the acquisition of ATP energy.

**[Table 14]**

| High-density liquid medium | 99%Glc | 9%C-Mix | 7%Glc | 7%C-Mix |
|---|---|---|---|---|
| Separate sterilization (1) | | | | |
| Calcium carbonate | 2.2% | 2.2% | 2.4% | 2.4% |
| Magnesium carbonate | 1.0% | 1.0% | 1.0% | 1.0% |
| Defatted germ koji | 3.0% | 3.0% | 3.0% | 3.0% |
| EBIOS tablets | 1.0% | 1.0% | 1.0% | 1.0% |
| Separate sterilization (2) | | | | |
| Glucose | 9.0% | 1.5% | 7.0% | 1.17% |
| Fructose | - | 1.5% | - | 1.17% |
| Mannose | - | 1.5% | - | 1.17% |
| Trehalose | - | 1.5% | - | 1.17% |
| N-Acetylglucosamine | - | 1.5% | - | 1.17% |
| Cellobiose | - | 1.5% | - | 1.17% |
| Separate sterilization (3) | | | | |
| HINUTE AM | 8.0% | 3.0% | 10% | 3.75% |
| Polypeptone | - | 3.0% | - | 3.75% |
| Lab-Lemco powder | - | 2.0% | - | 2-5% |
| Bonito extract-Exp Y | 1.0% | 1.0% | 1.0% | 1.0% |
| Magnesium glycerophosphate | 0.5% | 0.5% | 0.5% | 0.5% |
| Sodium citrate dihydrate | 0.72% | 0.72% | 0.72% | 0.72% |
| Rice germ fermentation extract | 2.0% | 2.0% | 2.0% | 2.0% |
| Heme iron | 0.001% | 0.001% | 0.001% | 0.001% |
| Beef fat | 0.056% | 0.056% | 0.056% | 0.056% |
| Polysorbate 20 | 0.169% | 0.169% | 0.169% | 0.169% |

**[Table 15]**

| | | Culture conditions | | | Turbidity | pH | Increase in electric conductivity | No. of cells achieved | Heat tolerance(%) | Survival rate after freezing and thawing(%) | Heat tolerance after freezing and thawing(%) |
|---|---|---|---|---|---|---|---|---|---|---|---|
| No. | | Medium | Temperature | Aeration | (2N sulfuric acid) | | (mS/m) | (×10⁸cfu/ml) | (57°C·15min) | (-30°C) | (57°C·15min) |
| 37°C | | | | | | | | | | | |
| preculture | 1 | MRS | 37 | Static culture | 5.97 | 3.96 | 110 | 22 | 110 | 160 | 58 |
| | 2 | Ca-MRS | 37 | Shaking | 8.27 | 5.48 | 900 | 33 | 42 | 110 | 49 |
| | 3 | High-density liquid (9%Glc) | 37 | culture Shaking culture | 34.40 | 4.88 | 2040 | 120 | 76 | 100 | 92 |
| | 4 | High-density liquid (9%C-Mix) | 37 | Shaking culture | 36.50 | 4.92 | 2090 | 110 | 100 | 120 | 92 |
| 42°C | | | | | | | | | | | |
| main culture | 5 | MRS | 42 | Static culture | 6.46 | 3.95 | 60 | 22 | 73 | 86 | 79 |
| | 6 | Ca-MRS | 42 | Shaking culture | 8.00 | 5.42 | 850 | 36 | 50 | 44 | 44 |
| | 7 | High-density liquid (7%Glc) | 42 | Shaking culture | 40.00 | 4.80 | 2300 | 170 | 94 | 86 | 110 |
| | 8 | High-density liquid (7%C-Mix) | 42 | Shaking culture | 40.40 | 5.04 | 2460 | 190 | 74 | 74 | 100 |

**[Table 16]**

| | | Organic acid concentration in medium & culture solution (mg/ml medium - culture solution) | | | Change in medium organic acid concentration (µmol/ml culture solution) | | | Organic acid producibility (µmol/ΔA) | | |
|---|---|---|---|---|---|---|---|---|---|---|
| No, | | Lactic acid | Acetic acid | Citric acid | Lactic acid | Acetic acid | Citric acid | Lactic acid | Acetic acid | Citric acid |
| | 1 | 11.4 | 0.14 | -0.02 | 126 | 2.33 | -0.104 | 21.8 | 0.40 | -0.018 |
| 37°C | 2 | 12.6 | 2.07 | -0.04 | 139 | 34.4 | 0.208 | 17.6 | 4.35 | 0.026 |
| preculture | 3 | 35.6 | 3.14 | -0.74 | 395 | 52.2 | -3.85 | 16.3 | 2.15 | -0.1 59 |
| | 4 | 35.0 | 9.14 | -0.73 | 388 | 152 | -3.80 | 13.4 | 5.23 | -0.131 |
| | 5 | 10.4 | 4.08 | 0.08 | 116 | 67.9 | 0.417 | 18.5 | 10.8 | 0.066 |
| 42°C | 6 | 12.1 | 0.01 | -0.03 | 135 | 0.17 | -0.156 | 17.7 | 0.02 | -0.020 |
| main culture | 7 | 43.1 | 7.82 | -0.93 | 478 | 130 | -4.84 | 16.4 | 4.45 | -0.166 |
| | 8 | 16.8 | 22.3 | -0.73 | 186 | 370 | -3.80 | 6.15 | 12.2 | -0.1 26 |
| Initial MRS liquid medium | | 025 | 2.33 | 1.57 | | | | | | |
| Initial high-density liquid medium | | 0.10 | 0.23 | 4.75 | | | | | | |
| Molecular weight | | 90.08 | 60.1 | 192 | | | | | | |

**[Table 17]**

| 37°C preculture | | | | | | |
|---|---|---|---|---|---|---|
| pH | | | | | | |
| | | Incubation time (min) | | | | |
| No. | Solvent | 0 | 30 | 60 | 90 | 120 |
| 1 | Glc | 7.25 | 7.11 | 7.01 | 6.92 | 6.80 |
| | XYL | 7.07 | 6.92 | 6.81 | 6.70 | 6.64 |
| 2 | Glc | 7.25 | 7.21 | 7.13 | 7.07 | 7.03 |
| | XYL | 7.14 | 7.12 | 7.04 | 7.00 | 6.96 |
| 3 | Glc | 7.12 | 6.98 | 6.84 | 6.69 | 6.53 |
| | XYL | 6.88 | 6.85 | 6.74 | 6.71 | 6.63 |
| 4 | Glc | 7.05 | 6.86 | 6.65 | 6.40 | 6.15 |
| | XYL | 6.85 | 6.69 | 6.64 | 6.52 | 6.41 |

| Δ pH | | | | | | |
|---|---|---|---|---|---|---|
| | | Incubation time (min) | | | | |
| No. | Solvent | 0 | 30 | 60 | 90 | 120 |
| 1 | Glc | 0.00 | 0.14 | 0.24 | 0.33 | 0.45 |
| | XYL | 0.00 | 0.15 | 0.26 | 0.37 | 0.43 |
| 2 | Glc | 0.00 | 0.04 | 0.12 | 0.18 | 0.22 |
| | XYL | 0.00 | 0.02 | 0.10 | 0.14 | 0.18 |
| 3 | Glc | 0.00 | 0.14 | 0.28 | 0.43 | 0.59 |
| | XYL | 0.00 | 0.03 | 0.14 | 017 | 0.25 |
| 4 | Glc | 0.00 | 0.19 | 0.40 | 0.65 | 0.90 |
| | XYL | 0.00 | 0.16 | 0.21 | 0.33 | 0.44 |

**[Table 18]**

| 42°C main culture | | | | | | |
|---|---|---|---|---|---|---|
| pH | | | | | | |
| | | Incubation time (min) | | | | |
| No. | Solvent | 0 | 30 | 60 | 90 | 120 |
| 5 | Glc | 7.07 | 6.94 | 6.83 | 6.64 | 6.60 |
| | XYL | 7.01 | 6.90 | 6.68 | 6.55 | 6.50 |
| 6 | Glc | 7.29 | 7.20 | 7.00 | 6.87 | 6.84 |
| | XYL | 7.25 | 7.12 | 7.02 | 6.91 | 6.83 |
| 7 | Glc | 6.95 | 6.63 | 5.98 | 5.36 | 4.99 |
| | XYL | 6.76 | 6.55 | 6.33 | 6.25 | 6.07 |
| 8 | Glc | 6.99 | 6.68 | 6.11 | 5.75 | 5.44 |
| | XYL | 6.80 | 6.66 | 6.34 | 6.18 | 6.09 |

| Δ pH | | | | | | |
|---|---|---|---|---|---|---|
| | | Incubation time (min) | | | | |
| No. | Solvent | 0 | 30 | 60 | 90 | 120 |
| 5 | Glc | 0.00 | 0.13 | 024 | 0.43 | 0.47 |
| | XYL | 0.00 | 0.1 | 0.33 | 0.46 | 0.51 |
| 6 | Glc | 0.00 | 0.09 | 0.29 | 0.42 | 0.45 |
| | XYL | 0.00 | 0.19 | 023 | 0.34 | 0.42 |
| 7 | Glc | 0.00 | 0.32 | 0.97 | 1.59 | 1.96 |
| | XYL | 0.00 | 0.21 | 0.43 | 0.51 | 0.69 |
| 8 | Glc | 0.00 | 0.31 | 0.88 | 1.24 | 1.55 |
| | XYL | 0.00 | 0.14 | 0.46 | 0.62 | 0.71 |

## Claims

1. A method for producing a liquid medium for lactic acid bacteria comprising a mixture containing at least carbonates, germ koji, a nitrogen source, and a carbon source, the method comprising: mixing carbonates and germ koji, sterilizing the mixture while separately sterilizing the other medium components which include at least a nitrogen source and a carbon source; and mixing the aforementioned mixture with the other separately sterilized medium components.

2. A method for producing a liquid medium for lactic acid bacteria comprising at least carbonates (A), germ koji (B), a germ koji extract (C), a nitrogen source (D), and a carbon source (E), the method comprising:
sterilizing a mixture of carbonates (A) and germ koji (B) while separately sterilizing the other medium components which include at least a germ koji extract (C), a nitrogen source (D), and a carbon source (E); and mixing the aforementioned mixture with the other separately sterilized medium components.

3. The method for producing a liquid medium for lactic acid bacteria according to claim 1 or 2, wherein the aforementioned germ koji is prepared with a solid-state culture medium composed of at least one member of the group consisting of defatted rice germ, defatted wheat germ, and defatted corn germ.

4. The method for producing a liquid medium for lactic acid bacteria according to claim 1 or 2, wherein the carbonates are a mixture of calcium carbonate and magnesium carbonate.

5. The method for producing a liquid medium for lactic acid bacteria according to any one of claims 1 to 4, wherein the liquid medium further comprises fatty acid.

6. The method for producing a liquid medium for lactic acid bacteria according to claim 5, wherein the fatty acid is animal or plant fat or oil containing oleic acid or a component of such fat or oil.

7. The method for producing a liquid medium for lactic acid bacteria according to any one of claims 1 to 4, wherein the liquid medium further comprises a polysorbate that serves as a surfactant.

8. The method for producing a liquid medium for lactic acid bacteria according to any one of claims 1 to 4, wherein the liquid medium further comprises iron.

9. The method for producing a liquid medium for lactic acid bacteria according to claim 8, wherein the iron is heme iron.

10. A method for culturing lactic acid bacteria using a liquid medium obtained by the method for producing a liquid medium for lactic acid bacteria according to any one of claims 1 to 9, wherein, the liquid medium is also used for preculture.

11. A method for culturing lactic acid bacteria using a liquid medium obtained by the method for producing a liquid medium for lactic acid bacteria according to any one of claims 1 to 9, wherein the liquid medium is used in each step of the multi-stage culture.

12. A method for culturing lactic acid bacteria using a liquid medium obtained by the method for producing a liquid medium for lactic acid bacteria according to any one of claims 1 to 9, wherein the liquid medium is used in any one of the steps of the multi-stage culture.

13. A lactic acid bacteria powder, which is obtained by separating bacterial cells of lactic acid bacteria from a medium and collecting and drying the bacterial cells, the lactic acid bacteria being obtained by the culture method according to any one of claims 10 to 12.
